(19) Europäisches Patentamt
European Patent Office
Office européen des brevets

(11) **EP 1 457 258 A1**

(12) **EUROPEAN PATENT APPLICATION**
published in accordance with Art. 158(3) EPC

(43) Date of publication:
**15.09.2004 Bulletin 2004/38**

(51) Int Cl.[7]: **B01J 23/74**, B01J 23/89,
C07C 1/04

(21) Application number: **02783882.0**

(22) Date of filing: **25.11.2002**

(86) International application number:
**PCT/RU2002/000507**

(87) International publication number:
**WO 2003/053568 (03.07.2003 Gazette 2003/27)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **21.12.2001 RU 2001135572**

(71) Applicant: **INSTITUT KATALIZA IMENI G.K. BORESKOVA SIBIRSKOGO OTDELENIA ROSSIISKOI AKADEMII NAUK Novosibirsk, 630090 (RU)**

(72) Inventors:
• **ITENBERG, Izabella Shenderovna Novosibirsk, 630090 (RU)**
• **KIRILLOV, Valerii Alexandrovich Novosibirsk, 630090 (RU)**

• **KUZIN, Nikolai Alekseevich Novosibirsk, 630128 (RU)**
• **PARMON, Valentin Nikolaevich Novosibirsk, 630090 (RU)**
• **SIPATROV, Anatoly Gennadyevich Novosibirsk, 630090 (RU)**
• **KHASIN, Alexandr Alexandrovich Moscow, 113035 (RU)**
• **CHERMASHENTSEVA, Galina Konstantinovna Novosibirsk, 630090 (RU)**
• **YURYEVA, Tamara Mihailovna Moscow, 113035 (RU)**

(74) Representative: **Goddar, Heinz J., Dr. FORRESTER & BOEHMERT Pettenkoferstrasse 20-22 80336 München (DE)**

(54) **CATALYST AND METHOD FOR PRODUCING HYDROCARBONS AND THE OXYGEN-CONTAINING DERIVATIVES THEREOF OBTAINED FROM SYNGAS**

(57) The present invention relates to catalysts and methods for producing hydrocarbons, including liquid synthetic fuels, olefins, solid hydrocarbons, as well as oxygen-containing derivatives thereof (for example, alcohols) from a mixture of CO and hydrogen (syngas).

The problem to be solved by the present invention is to provide an effective catalyst and a process for the catalytic production of hydrocarbons and their oxygen-containing derivatives from synthesis gas with a high productivity per volume unit of a reactor.

The process is carried out by passing carbon monoxide and hydrogen through one or more stationary particles (bodies) of a concentrated permeable catalyst containing at least 0.4 g/cm³ of an assembly of phases comprising a phase of a catalytically active metal, fixed on a phase of a support of oxidic nature, producing an appreciable influence on the dispersity of the active metal phase or on other physicochemical properties thereof, wherein the catalyst body has a permeability of at least $5 \cdot 10^{-15}$ m². One of Group VIII metals or an intermetallic containing them is used as the catalytically active metal, wherein the content of the catalytically active metal phase in the assembly of the phases is at least 5 wt.%.

**EP 1 457 258 A1**

**Description**

Field of the Art

The present invention relates to catalysts and methods for producing hydrocarbons, including liquid synthetic fuels, olefins, solid hydrocarbons, as well as oxygen-containing derivatives thereof (for example, alcohols) from a mixture of CO and hydrogen (synthesis gas or "syngas"). Subsequently, the produced hydrocarbons can be used for generating energy (i.e., for combusting as fuel) or for obtaining useful chemical compounds (for example, hydrocarbons having a smaller number of carbon atoms per molecule, polymeric materials, higher alcohols, surfactants, etc.).

Description of the Related Art

Known in the art are methods for converting syngas into valuable chemical products by the reactions:

$$nCO + (2n+1)H_2 = C_nH_{2n+2} + nH_2O$$

$$nCO + (2n)H_2 = C_nH_{2n} + nH_2O$$

$$nCO + (2n)H_2 = C_nH_{2n+1}OH + (n-1)H_2O$$

in the presence of a catalyst. These methods are united under the name of "Fischer-Tropsch synthesis". The Fischer-Tropsch synthesis enables quantitative production and isolation of saturated and unsaturated hydrocarbons having any number of carbon atoms from 1 (methane) to more than 100, as well as alcohols. The catalyst usually comprises one or more elements from the group: iron, cobalt, nickel, ruthenium. The $CO:H_2$ ratio in syngas may vary depending on the method for producing thereof, and the mixture may also be diluted with nitrogen.

A distinctive feature of the Fischer-Tropsch synthesis is that the process is essentially exothermic, in combination with the process selectivity and activity being highly temperature-sensitive. An increase in the temperature leads to an increase in the reaction rate, but shifts the process selectivity toward the formation of light hydrocarbons and methane, this being undesirable. Therefore, a necessary condition for an optimal course of the Fischer-Tropsch synthesis is maintaining a prescribed temperature and providing iso-thermicity of reactor.

Another specific feature of the Fischer-Tropsch synthesis is a relatively low rate of the process. Thus, the specific reaction rate when using Co-Ru-TiO$_2$-containing catalysts under conditions proximate to industrial ones (20 atm, 40% CO conversion) is 220 kg of hydrocarbons per cubic meter of catalyst per hour (US 4670475, C07C 1/04, 06.02.1987). It follows therefrom that lowering the catalyst content per unit reaction volume is undesirable, because it leads to lowering the reactor productivity and to increasing the overall dimensions of industrial apparatus.

Numerous types of Fischer-Tropsch synthesis reactors are known: conventional tubular-type reactors, three-phase slurry reactors, fluidized bed reactors, and others.

In tubular reactors stationary catalyst particles (granules) are usually used, having a size less than 20 mm, arranged stationary in cylindrical tubes. These particles may have various shapes (trilobes, spheres, cylinders) and contain voidages and pores whose volume is from 30 to 50% of the geometrical volume of the particles. A flow of reagents passes through the catalyst bed, flowing around each particle. One of the disadvantages of fixed bed reactors is low radial thermal conductivity in the catalyst bed. For a radial temperature profile of 5°C to be provided, the diameter of the tubes cannot be very large and usually does not exceed 6—10 cm. At the same time, because of pore-diffusion resistance phenomena in the catalyst particles, high productivity and selectivity of the process can be achieved only with the catalyst particle size being less than 0.5 mm. The hydrodynamic resistance of such a reactor is very high. Moistening of catalyst particles with reaction products inevitably leads to the coalescence of catalyst particles and to the formation of "dead" zones simultaneously with sufficiently broad gas-filled channels. As a result, the effectiveness of using the catalytically active component proves to be low. In the case of using sufficiently large catalyst particles (over 5 mm), the process is characterized by a high yield of methane and a low selectivity. This is the consequence of a difference in the diffusion coefficients of the reagents, which leads to enriching the internal space of the catalyst particle with hydrogen and contributes to a higher rate of formation of light hydrocarbons (first of all of methane) compared with the target products.

A possible way for overcoming the discussed contradiction is to use large (over 0.5 mm) catalyst particles comprising a porous support and a catalytically active component applied to the support in such a manner that the main mass of the catalytically active component is concentrated in a thin surface layer of the support. In the internal space of the support the catalytically active component is absent. A disadvantage of such "egg-shell" catalysts is complexity of their manufacture. Besides, with the use of "egg-shell" catalysts the concentration of the catalytically active component in the reaction volume is not high, this lowering the process capacity and leading to an increase in the overall dimensions of the reactor.

In slurry reactors use is made of a slurry of catalyst particles smaller than 100 μm. A flow of reagents passes through the slurry in the form of dispersed bubbles. In this case the pore diffusion processes do not essentially affect the rate and selectivity of the catalytic reaction. Slurry reactors are also advantageous in being isothermic. However, the productivity of slurry apparatus remains low because of the catalytically active component concentration in the slurry being limited to not over 0.2 $g/cm^3$ (for the necessary dynamic viscosity to be provided, the slurry should contain more than 20—25 wt.% of catalyst particles). Besides, the rate of the catalytic reaction may be limited by mass transfer processes at the gas-liquid interface. An additional disadvantage of slurry reactors is that the regime realized in slurry reactors is close to the regime of perfect mixing, whereby the effectiveness of using the catalyst and the process selectivity are lowered, compared with the perfect displacement regime typical of fixed bed reactors. Finally, the use of slurry reactors requires incorporating a technically complicated step of separating reaction products from catalyst particles into the process flowsheet.

In US 5786393, C07C 27/00, 28.07.1998, for raising the process effectiveness it is proposed to use recirculation of the liquid phase through the fixed bed catalyst. The reactor of such type was called "trickle bed" reactor. Its distinctive feature is simultaneous supply through the catalyst bed of a reaction gaseous mixture and an inert liquid (preferably either as an upflow or downflow). As a result of such organization of mass flows, it becomes possible to raise both the productivity and selectivity of the process owing to better mass transfer at the gas-liquid interface, provision of effective heat removal from the catalyst bed by the inert liquid flow and lowering of longitudinal mixing. However, trickle reactors suffer from a number of essential disadvantages. First of all, this is the presence of hydrodynamic heterogeneities in the catalyst bed, and also a large pressure differential on the catalyst bed at large gas and liquid velocities, which is associated with a low porosity of fixed bed reactors (usually less than 45%). Besides, like in the case of the earlier described types of Fischer-Tropsch synthesis reactors with fixed bed catalysts, pore-diffusion resistance phenomena influence essentially the effectiveness of using the active catalyst component, lowering thereby the productivity of the process.

In US 6211255, C07C 27/00, 03.04.2001 it is proposed to use a catalyst applied to a monolithic support having parallel discrete channels. US 6211255 teaches and discusses the use of extended monolith supports (for example, having a length greater than 10 cm) with an active component applied to the surface of the channels. The authors of said invention propose to use supports with 100 to 1,000 channels per square inch (15 to 155 channels per square centimeter). As the gas flows through a liquid-filled monolithic catalyst, a Taylor flow regime (or slug flow) takes place, which, in the opinion of the authors of said invention, promotes high mass transfer at the gas-liquid interface. An advantage of the proposed process is that the degree of catalyst utilization is high and the hydraulic resistance of the monolithic catalyst is relatively low. The proposed process is disadvantageous in that the catalytically active substance is diluted appreciably with the support, and the proportion of the reaction volume occupied by the catalyst is small. Thus, in the examples presented in the cited invention the content of the catalytically active component ($CoRe/Al_2O_3$) does not exceed 0.1 g per cubic centimeter of the monolithic catalyst. Therefore, like in the case of slurry reactors, the productivity per unit volume of the monolithic catalyst reactor is essentially limited by the small concentration of the catalytically active substance in the reaction volume. Besides, an essential disadvantage of the invention under discussion is the necessity of circulating the liquid for effective removal of the heat evolving in the course of the reaction.

The prior art most relevant to the present invention is a process for the conversion of synthesis gas, proposed in US 6262131, C07C 027/00, B01J 023/02, 2001, with the use of a structured catalyst system. A distinctive feature of the cited system is that synthesis gas (or a liquid saturated with synthesis gas or a gas-liquid flow) is passed through a structured Fischer-Tropsch synthesis catalyst which has a voidage ratio of at least 45% and provides passage of the gas (liquid or gas-liquid) flow in a regime when Taylor flow cannot be formed. The gas flow through liquid-filled channels occurs in a substantially turbulent regime of individual gas bubbles. According to the text of the cited US Patent, for this to take place, a mean length/diameter ratio (L/D) of flow paths must be less than 100, preferably less than 10. The characteristic diameter of the flow paths (channels) in the text of the cited patent is indicated to be 1.5 mm with the length less than 150 mm. The authors of the cited invention believe that this provides better mass transport inside the channels and lowers the probability of laminar flow zones being formed. For increasing the productivity of the catalyst volume, its content should be no less than 10% of the reactor volume. An appreciable dilution of the catalytically active component with the support should be regarded as one of the disadvantages of the known process.

Summary of the Invention

The problem to be solved by the present invention is to provide an effective catalyst and a process for the catalytic production of hydrocarbons and their oxygen-containing derivatives from synthesis gas with a high productivity per volume unit of a reactor. For this to be achieved, the catalyst and the process should meet the following requirements:

1. High concentration of the catalytically active component in the reaction volume;
2. High effectiveness of using the catalytically active component;
3. Provision of temperature homogeneity of the catalyst bed.

In the present invention it is proposed to carry out the process of synthesis gas conversion into hydrocarbons by passing carbon monoxide and hydrogen through one or more stationary particles (bodies) of a concentrated permeable catalyst. It is preferable that one of the linear dimensions of the catalyst body should be comparable with (i.e., should be no less than 10% of) the minimum linear size of the reactor.

The term "concentrated" means a high concentration of the catalytically active component in the catalyst body, i.e., at least 0.4 $g/cm^3$ of the catalyst body, preferably higher than 0.8 $g/cm^3$. The term "catalytically active component" is understood here as an assembly of phases, including the phase of an active metal (for example, of cobalt, iron, nickel, ruthenium or intermetallic compounds with their content), fixed on the phase of the support of active nature, having decisive influence on the physicochemical properties of the active metal phase (for example, on its dispersity). The content of active metal in said assembly of phases must be greater than 5 wt.%, preferably 8 to 30 wt.%.

The term "permeable" implies that the catalyst body has a permeability of at least $5 \cdot 10^{-15}$ $m^2$, preferably greater than $10^{-13}$ $m^2$.

Such permeability in combination with high effectiveness of using the catalytically active metal can be attained on condition that the volume of pores having a size smaller than 70 μm is at least 90% of the pore volume of the catalyst. It is preferable that the pore volume of the concentrated permeable catalyst should be at least 40% of the geometrical volume of the concentrated permeable catalyst body.

An essential feature of the proposed process is that it is proposed to pass the flow of carbon monoxide and hydrogen through each body of the concentrated permeable catalyst along stochastically distributed transport pores having a characteristic size greater than 1 μm. Flowing around one or more concentrated permeable catalyst bodies with a flow of reagents is undesirable, because this lowers effectiveness factor of using the active component. The geometrical form of the concentrated permeable catalyst body may be any, and it depends on the method of preparing and by the requirements to be met by a particular reactor. The most preferable forms are plates (including disks) and hollow cylinders with various geometry of cross-sections (including hollow cylinders of revolution). In the case of cylindrical shape, the flow of reagents can be directed from the external geometrical surface inwards or vice versa. The thickness of a plate (or cylinder wall) may be from fractions of a millimeter to 1 meter; an optimal size is determined by the technical parameters of the process and the condition of attaining reasonable pressure drop on the catalyst body.

The microscopic size of the transport pores of the concentrated permeable catalyst makes it possible to organize forced convective motion of the flow containing carbon monoxide and hydrogen through the transport pores of the catalyst, moistened with a liquid (including products of the Fischer-Tropsch synthesis)in the so-called "film" or "annular" regime, when the surface of the gas-liquid interface is maximum and approaches the surface of the transport pores. In such regime the mass transport processes are substantially intensified owing to the developed surface of the interface. Besides, longitudinal mass transport in a direction opposite to the movement of the flow is negligibly small, and therefore the catalytically active component can be used more effectively. It is desirable that the volume of transport pores (pores larger than 1 μm) should be greater than 25% and not greater than 70% of the geometrical volume of the concentrated permeable catalyst body.

The term "stationary" implies that catalyst bodies do not move relative to each other and to the reactor body. It is allowable that catalyst bodies perform periodic oscillations ("jiggle") because of reactor vibrations and variations in the flow rate of the reagents.

The invention contemplates the possibility of arranging in the reaction volume several concentrated permeable catalyst bodies either parallel or sequentially to the flow of CO and $H_2$. It is preferable that one of the linear dimensions of the catalyst bodies be comparable with (i.e., should be no less than 20% of) the minimum linear dimension of the reactor.

Besides, in the present invention it is proposed to carry out the process of synthesis gas conversion into hydrocarbons by passing carbon monoxide and hydrogen through concentrated permeable catalyst bodies having a thermal conductivity no lower than 1 $W \cdot m^{-1} \cdot K^{-1}$.

An increased thermal conductivity of the catalyst bodies can be achieved by introducing into the composition of the concentrated permeable catalyst the phase of a metal inert under the reaction conditions of Fischer-Tropsch synthesis (for example, of aluminum, zinc, copper, their alloys, and others) or a graphite-like phase (for example, porous carbon, catalytic fibrous carbon, nanotubes). A sufficient thermal contact must be provided between the grains of a metal (or graphite-like phase). Metal grains may have an arbitrary shape (a sphere, a hollow sphere, wire, a perforated plate, sawings, etc.) and size, providing the possibility of preparing concentrated permeable catalyst bodies with the claimed parameters.

An enhanced thermal conductivity of the concentrated permeable catalyst bodies makes it possible to lower the temperature gradient inside the catalyst, i.e., to provide the process running in a regime proximate to isothermal. The removal of heat from the concentrated permeable catalyst bodies can be provided by thermal contact of the catalyst bodies with the reactor wall or with a wall of additional heat exchange devices introduced into the reaction volume.

It is necessary to note that the invention implies the possibility of combined use of a concentrated permeable catalyst together with other catalysts which do not have the claimed parameters. For instance, a concentrated permeable catalyst body can be used as a catalytically active distributor of a gas flow, as a heat exchange device, and other auxiliary devices. A distinctive feature of the present invention in such a case is that at least part of carbon monoxide interacts with hydrogen in the step of flowing through the stationary body of the concentrated permeable catalyst.

Another additional advantage of the proposed process is the simplicity of separating the reaction products from the concentrated permeable catalyst and the absence of mechanical admixtures (dust) in the composition of the products.

Still another additional advantage of the proposed process is the possibility of arranging the reactor containing concentrated permeable catalyst bodies both vertically and horizontally, as well as at any required angle to the vertical. This makes it possible to locate the reactor on any movable systems, including floating platforms.

Yet another additional advantage of the proposed invention is the possibility of using one or more concentrated permeable catalyst bodies as a compact module; an apparatus having an arbitrary productivity can be assembled of several such modules. It is preferable that one of the linear dimensions of the catalyst bodies should be comparable with (i.e., should be no less than 20% of) the minimum linear dimension of the module. Additional modules can also be added to an already acting apparatus without stopping the process.

The invention is illustrated by the following drawings and examples:

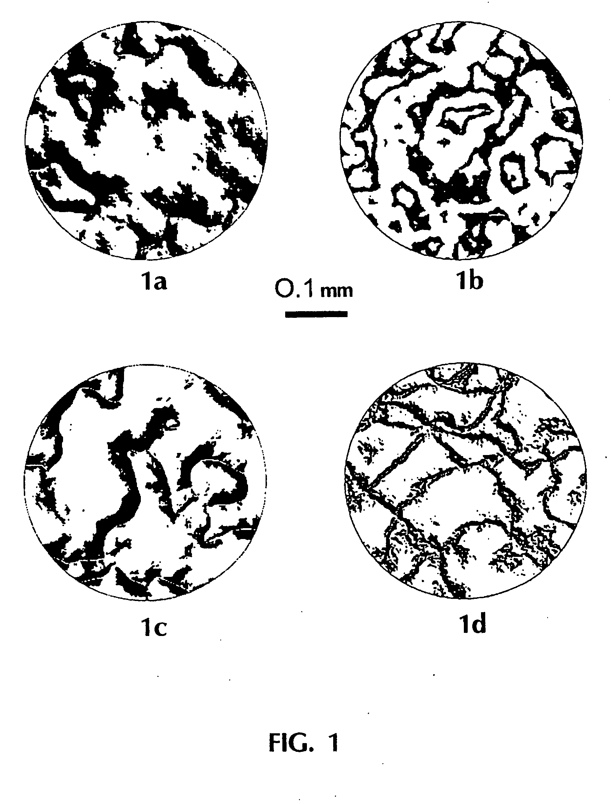

**Figure 1** illustrates some possible variants of the disposition of transport pores inside concentrated permeable catalyst bodies (diagrammatic representations of catalyst body section).

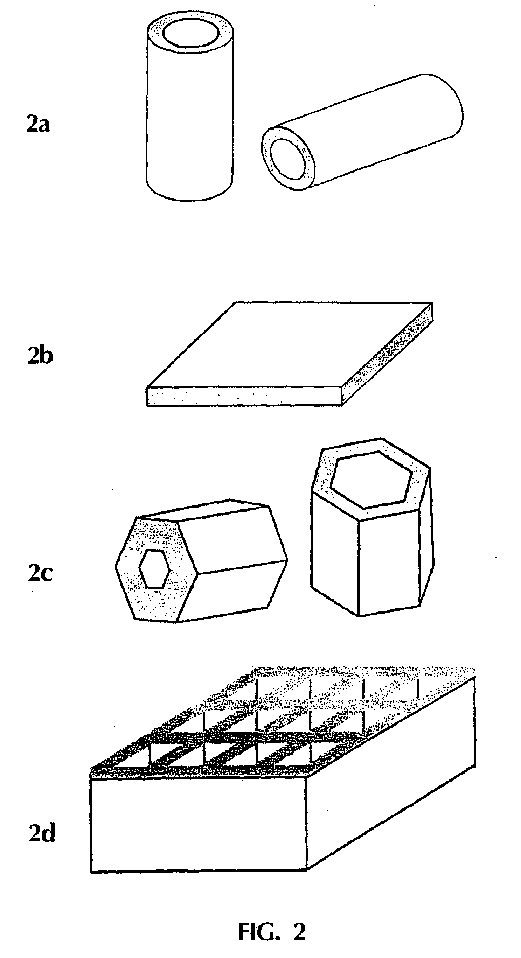

**Figure 2** illustrates some possible variants of the geometry of concentrated permeable catalyst bodies.
**Figure 3** illustrates some possible variants of the disposition of concentrated permeable catalyst bodies in a reactor and relative to a flow of reagents and reaction products (white arrows indicate the flow of reagents; black arrows indicate the flow of reaction products).

## Example 1

The process for catalytic conversion of synthesis gas into hydrocarbons is carried out by passing a gas flow containing 20 vol.% of carbon monoxide, 40 vol.% of hydrogen, 6 vol.% of nitrogen and saturated vapors of n-tetradecane (34 vol.%) sequentially through two concentrated permeable catalyst bodies at T = 210°C. A first body is a 5.0 mm thick disk having a circular section of 15.7 in diameter; a second body is a 4.4 mm thick disk having a circular section of 15.8 in diameter; each body contains 0.9 g/cm$^3$ of an assembly of phases comprising a phase of metallic cobalt fixed on an aluminum phase. The content of the metallic cobalt phase in said assembly of phases is 24 wt.%. The dependence of pressure drop on the bodies on the gas flow passing therethrough at the process temperature (210°C) is described by the equation P(atm) = $3.7 \cdot 10^5$ V(m$^3$/s), this corresponding to the mean permeability K = $1.6 \cdot 10^{-14}$ m$^2$. Investigations of the porous structure of the concentrated permeable catalyst bodies have shown that the volume of catalyst pores is 45% of the geometrical volume of the body, 98% of the volume accounting for pores having a size smaller than 70 μm, the characteristic size of transport pores being 6—7 μm.

The productivity of the process is about 1.2 mmole of CO per hour per cubic centimeter of the geometrical volume of the catalyst body at P (CO + H$_2$) = 0.6 atm, T = 210°C, the degree of CO conversion 8—22%. The value of the parameter α of the Anderson-Schulz-Flory distribution is about 0.78 for the products of the fraction of saturated hydrocarbons.

## Example 2

The process of catalytic conversion of synthesis gas into hydrocarbons is carried out by passing a gas flow containing 20 vol.% of carbon monoxide, 40 vol.% of hydrogen, 6 vol.% of nitrogen and saturated vapors of n-tetradecane (34 vol.%) through one concentrated permeable catalyst body at T = 210°C. The body is a 5.2 mm thick disk having a circular section of 16 in diameter and contains 0.9 g/cm$^3$ of an assembly of phases comprising a phase of metallic nickel fixed on a phase of magnesium silicate. The content of the metallic nickel phase in said assembly of phases is 22 wt.%. The dependence of pressure drop on the body on the gas flow passing therethrough at the process temperature (210°C) is described by the equation P(atm) = $9 \cdot 10^4$ V(m$^3$/s), this corresponding to the mean permeability K = $3.6 \cdot 10^{-14}$ m$^2$. Investigations of the porous structure of the concentrated permeable catalyst body have shown that the volume of catalyst pores is 48% of the geometrical volume of the body, 96% of the volume accounting for pores having a size smaller than 70 µm, the characteristic size of transport pores being 6—7 µm.

The productivity of the process is about 1.4 mmole of CO per hour per cubic centimeter of the geometrical volume of the catalyst body at P (CO + H$_2$) = 0.6 atm, T = 210°C, the degree of CO conversion 8—22%. The value of the parameter $\alpha$ of the Anderson-Schulz-Flory distribution is about 0.38 for the products of the fraction of saturated hydrocarbons. (The catalyst can be used for processes of converting synthesis gas into methane and light hydrocarbons).

**Example 3 (comparative to Examples 4—6)**

The process for catalytic conversion of synthesis gas into hydrocarbons is carried out by passing a gas flow containing 20 vol.% of carbon monoxide, 40 vol.% of hydrogen, 6 vol.% of nitrogen and saturated vapors of n-tetradecane (34 vol.%) through a slurry of Co-Al catalyst particles having a size smaller than 140 µm in n-tetradecane. The content of the catalyst in the slurry is 20 wt.% The catalyst comprises a phase of metallic cobalt fixed on a phase of anionically modified cobalt aluminate. The gas flow is organized in the form of separate disperse bubbles having a size less than 0.2 mm. The bubble contact time in the slurry is larger than 4 sec. Under such conditions the mass transport at the gas-liquid interface does not limit the velocity of the process. The slurry is intensively stirred mechanically.

The results of catalytic tests carried out at the pressure of the reaction mixture of 1 atm, T = 210°C are presented in Table 1.

**Example 4**

The process for catalytic conversion of synthesis gas into hydrocarbons is carried out by passing a gas flow containing 20 vol.% of carbon monoxide, 40 vol.% of hydrogen, 6 vol.% of nitrogen and saturated vapors of n-tetradecane (34 vol.%) through one concentrated permeable catalyst body at T = 210°C. The body is a 6.2 mm thick disk having a circular section of 15 in diameter and contains 1.0 g/cm$^3$ of an assembly of phases identical with the catalyst used in Example 3, i.e., comprising a phase of anionically modified cobalt aluminate. The content of the metallic cobalt phase in said assembly of phases is 28 wt.%. For the provision of high thermal conductivity, the concentrated permeable catalyst also contains in its composition a phase of crystalline copper. The thermal conductivity of the concentrated permeable catalyst body is determined experimentally to be about 5 W/m/K. The dependence of pressure drop on the body on the gas flow passing therethrough at the process temperature (210°C) is described by the equation P(atm) = 3.6·10$^4$ V(m$^3$/s), this corresponding to the permeability K = 1.2·10$^{-13}$ m$^2$. Investigations of the porous structure of the concentrated permeable catalyst body have shown that the volume of catalyst pores is 62% of the geometrical volume of the body, 97% of the volume accounting for pores having a size smaller than 70 µm, the characteristic size of transport pores being 10—12 µm.

The results of catalytic tests carried out at a pressure of the reaction mixture of 1 atm, T = 210°C, are presented in Table 1. A comparison of the obtained experimental data with the data of testing catalytically active catalyst particles in a laboratory slurry reactor (see Example 3) has shown that the degree of the catalytically active component utilization is 100%. The value of the Anderson-Schulz-Flory parameter, expressing the selectivity of the process with respect to heavy hydrocarbons, is the same for the process on the concentrated permeable catalyst and for tests on the suspended catalytically active component used in the catalyst. The selectivity with respect to unsaturated hydrocarbons for the proposed process is substantially higher than the corresponding selectivity of the process with the use of the suspended catalytically active component, this reflecting a more effective transport of products from the surface of the catalyst into the gaseous phase.

**Example 5**

The process is carried out as in Example 4, but the porous structure of the catalyst body in the absence of gas flow (before the commencement of tests) is filled with liquid, namely with n-tetradecane. The catalyst body is a 4.6 mm thick disk having a circular section of 15 mm in diameter. The concentrated permeable catalyst contains 0.8 g/cm$^3$ of a assembly of phases identical with the catalyst used in Example 3, that is, comprising a phase of metallic cobalt, fixed on the phase of anionically modified cobalt aluminate. The content of the metallic cobalt phase in the mentioned assembly is 28 wt.%. For providing high thermal conductivity, the concentrated permeable catalyst also contains in its composition a phase of metallic copper. The thermal conductivity of the concentrated permeable catalyst body is experimentally determined to be about 5 W/m/K. Investigations of the porous structure of the concentrated permeable catalyst body have shown that the volume of catalyst pores is 58% of the geometrical volume of the body, 99% of the volume accounting for pores having a size smaller than 70 µm. The dependence of the pressure drop on the catalyst body on the gas flow passing therethrough at the process temperature (210°C) is described by the equation P(atm) = 0.141 + 4.5·$10^4$ V($m^3$/s), this corresponding to the permeability K = 0.72·$10^{-13}$ $m^2$ and to the characteristic size of pores 7.6 μm.

The results of catalytic tests carried out at a pressure of 1 atm, T = 210°C, are presented in Table 1. A comparison of the obtained experimental data with the data of testing catalytically active catalyst particles in a laboratory slurry reactor (see Example 1) shows that the degree of the catalytically active component utilization is 70%. The value of the Anderson-Schulz-Flory parameter, expressing the selectivity of the process with respect to heavy hydrocarbons, is the same for the process on the concentrated permeable catalyst and for tests on the suspended catalytically active component used in the catalyst. The selectivity with respect to unsaturated hydrocarbons for the proposed process is substantially higher than the corresponding selectivity of the process with the use of the suspended catalytically active component, this reflecting a more effective transport of products from the surface of the catalyst into the gaseous phase.

### Example 6

The process is carried out as in Example 3, but concurrently with a gas flow a liquid flow of n-tetradecane is passed through the concentrated catalyst body. The ratio of the space velocities of the flows is $V_{gas}/V_{liquid}$ = 50.

The results of catalytic tests carried out at a pressure of 1 atm, T = 210°C, are presented in Table 1. A comparison of the obtained experimental data with the data of testing catalytically active catalyst particles in a laboratory slurry reactor (see Example 1) shows that the degree of the catalytically active component utilization is 68%. The value of the Anderson-Schulz-Flory parameter, expressing the selectivity of the process with respect to heavy hydrocarbons, is the same for the process on the concentrated permeable catalyst and for tests on the suspended catalytically active component used in the catalyst. The selectivity with respect to unsaturated hydrocarbons for the proposed process is substantially higher than the corresponding selectivity of the process with the use of the suspended catalytically active component, this reflecting a more effective transport of products from the surface of the catalyst into the gaseous phase.

The Examples presented above show that the productivity per unit volume of the reactor with the use of the concentrated permeable catalyst can reach 10—16 kg of hydrocarbons per cubic meter of the reactor at a partial pressure of synthesis gas being 0.6 atm (Examples 4—6), this exceeding essentially the productivity of the slurry reactor (example 3).

### Example 7

Similarly to Example 3, the concentrated permeable catalyst contains 0.6 g/$cm^3$ of an assembly of phases, comprising a phase of metallic cobalt fixed on a phase of anionically modified zinc aluminate. The content of the metallic cobalt phase in the catalytically active component is 14 wt.%. For providing high thermal conductivity, the concentrated permeable catalyst also contains in its composition a phase of metallic aluminum. The thermal conductivity of the concentrated permeable catalyst is experimentally determined to be about 3 W/m/K. The dependence of the pressure drop on the catalyst body on the gas flow passing therethrough at the process temperature (210°C) is described by the equation P(atm) = 0.076 1.6·$10^4$ V($m^3$/s), this corresponding to the permeability K = 2·$10^{-13}$ $m^2$. Investigations of the porous structure of the concentrated permeable catalyst body have shown that the volume of catalyst pores is 58% of the geometrical volume of the body, 95% of the volume accounting for pores having a size smaller than 70 μm, the characteristic size of transport pores being 15—20 μm.

The results of catalytic tests carried out at a pressure of the reaction mixture of 1 atm, T = 210°C, are presented in Table 1. A comparison of the obtained experimental data with the data of testing catalytically active catalyst particles in a laboratory slurry reactor (see Example 1) has shown that the degree of the catalytically active component utilization is 75%. The value of the Anderson-Schulz-Flory parameter, expressing the selectivity of the process with respect to heavy hydrocarbons, is the same for the process on the concentrated permeable catalyst and for tests on the suspended catalytically active component used in the catalyst. The selectivity with respect to unsaturated hydrocarbons for the proposed process is substantially higher than the corresponding selectivity of the process with the use of the suspended catalytically active component, this reflecting a more effective transport of products from the surface of the catalyst into the gaseous phase.

### Example 8

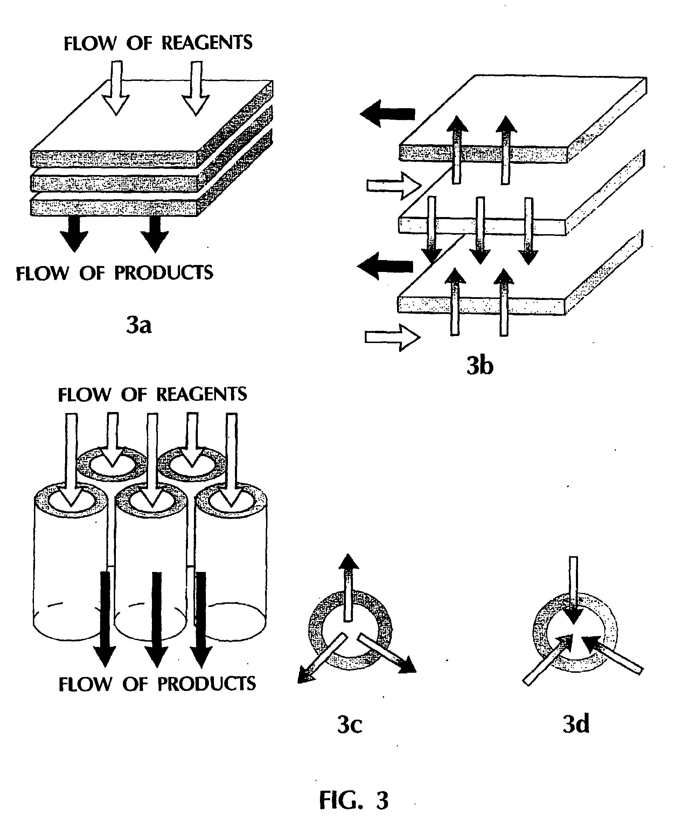

Similarly to Example 3, the concentrated permeable catalyst contains 0.9 g/$cm^3$ of an assembly of phases comprising a phase of metallic cobalt fixed on a phase of anionically modified magnesium aluminate. The content of the metallic cobalt phase in the catalytically active component is 22 wt.%. The catalyst body is a hollow cylinder of revolution having an internal diameter of 8 mm, an external diameter of 17 mm and a height of 12 mm. A flow of reagents is supplied into the cylinder interior from one of its ends, the opposite end of the cylinder interior being plugged. Further, the flow of the reagents passes radially through the cylinder wall toward its external geometrical surface (see Figure 3c). For providing high thermal conductivity, the concentrated permeable catalyst contains in its composition graphite-like carbon phase comprising a three-dimensional carbon matrix formed by banded layers of carbon having a thickness of 0.01—1 µm and a radius of curvature of 0.01—1 µm, characterized by a porous structure with a pore distribution having a maximum in the range of 0.02—0.2 µm (US 4978649, C01B 31/10, 1990). The thermal conductivity of the concentrated permeable catalyst is determined experimentally to be about 1.2 W/m/K. The dependence of the pressure drop on the catalyst body on the gas flow passing therethrough at the process temperature (210°C) is described by the equation $P(atm) = 0.07 + 1.9 \cdot 10^4 \, V(m^3/s)$, this corresponding to the permeability $K = 1.2 \cdot 10^{-13} m^2$. Investigations of the porous structure of the concentrated permeable catalyst bodies have shown that the volume of catalyst pores is 57% of the geometrical volume of the body, 92% of the volume accounting for pores having a size smaller than 70 µm, the characteristic size of transport pores being 17—22 µm.

The productivity of the process is about 0.7 mmole of CO per hour per cubic centimeter of the geometrical volume of the catalyst body at $P (CO + H_2) = 0.6$ atm, T = 210°C, the degree of CO conversion 7—20%. On conversion to unit of the reaction volume (i.e., taking into account the volume of the cylinder interior), the productivity of the process is about 0.55 mmole of CO per cubic centimeter of the reaction volume per hour.

Table 1

| Example No. | Rate of gas flow, lit./hr | Degree of CO conversion, % | Rate of CO conversion, mmole/hour | | Rate of methane conversion, mmole/hour | | α parameter of ASF for paraffin fraction | Propylene/propane yield ratio, $C_3$ =/- |
|---|---|---|---|---|---|---|---|---|
| | | | per g of $Co^0$ | per $cm^3$ of reactor | per g of $Co^0$ | per $cm^3$ of reactor | | |
| 3 (comparison) | 0.92 | 11.7 | 5.3 | 0.3 | 0.3 | 0.01 | 0.83 | 2.0 |
| | 0.65 | 14.0 | 5.2 | 0.3 | 0.3 | 0.01 | 0.83 | 2.0 |
| 4 | 0.94 | 11.5 | 5.3 | 1.6 | 0.3 | 0.13 | 0.82 | 4.8 |
| | 0.68 | 14.6 | 5.3 | 1.6 | 0.5 | 0.14 | 0.80 | 4.9 |
| 5 | 0.93 | 5.1 | 3.2 | 0.7 | 0.4 | 0.08 | 0.79 | 3.7 |
| | 0.67 | 9.0 | 4.0 | 1.0 | 0.5 | 0.12 | 0.80 | 2.8 |
| 6 | 0.94 | 4.9 | 3.1 | 0.7 | 0.3 | 0.08 | 0.82 | 4.0 |
| | 0.64 | 8.7 | 3.8 | 0.9 | 0.4 | 0.11 | 0.80 | 3.3 |
| 7 | 0.92 | 4.2 | 1.9 | 0.4 | 0.07 | 0.03 | 0.82 | 3.8 |
| | 0.67 | 5.6 | 2.1 | 0.4 | 0.08 | 0.02 | 0.81 | 3.6 |

EP 1 457 258 A1

**Claims**

1. A catalyst for producing hydrocarbons and/or oxygen-containing derivatives thereof from synthesis gas, comprising at least 0.4 g/cm$^3$ of an assembly of phases, comprising as phase of a catalytically active metal, fixed on a phase of a support of an oxidic nature, producing an appreciable influence on the dispersity of the active metal phase or on other physicochemical properties thereof, wherein the catalyst body has a permeability of at least 5·10$^{-15}$ m$^2$.

2. A catalyst according to claim 1, wherein one of Group VIII metals or an intermetallic containing them is used as the catalytically active metal.

3. A catalyst according to claims 1—2, wherein the content of the catalytically active metal phase in the assembly of the phases is at least 5 wt.%.

4. A catalyst according to claims 1—3, wherein the volume of pores having a size smaller than 70 μm is at least at least 90% of the total pore volume of the catalyst body.

5. A catalyst according to claims 1—4, wherein its composition comprises a phase of a metal inert to synthesis gas and/or a graphite-like phase.

6. A catalyst according to claim 5, wherein copper and/or zinc and/or aluminum and/or their alloys are used as the metal inert to synthesis gas.

7. A catalyst according to claims 1—6, wherein the body thereof has a thermal conductivity of at least 1 W·m$^{-1}$·K$^{-1}$.

8. A catalyst according to claims 1—7, wherein at least part of the volume of its pores is filled with hydrocarbons and/or with their oxygen-containing derivatives in liquid or solid aggregate state.

9. A process for producing hydrocarbons and/or oxygen-containing derivatives thereof, comprising as one of steps passing a gas flow containing synthesis gas through one or more bodies of a concentrated permeable catalyst according to any of claims 1—8.

10. A process according to claim 9, wherein one of the linear dimensions of the concentrated permeable catalyst amounts to no less than 20% of the minimum linear size of the reactor.

11. A process according to claims 9—10, according to which the concentrated permeable catalyst bodies completely overlap the gas flow.

12. A process according to claims 9—10, according to which a flow of a liquid phase is passed through the concentrated permeable catalyst bodies cocurrently with passing a gas flow, said liquid phase making up a single gas-liquid flow with said gas flow.

13. A process according to claims 9—12, wherein the step of passing a gas flow or a gas-liquid flow through the concentrated permeable catalyst bodies is reiterated.

1a     O.1 mm     1b

1c     1d

FIG. 1

2a

2b

2c

2d

FIG. 2

FLOW OF REAGENTS

FLOW OF PRODUCTS

**3a**

**3b**

FLOW OF REAGENTS

FLOW OF PRODUCTS

**3c**

**3d**

FIG. 3

## INTERNATIONAL SEARCH REPORT

| | |
|---|---|
| | International application No. |
| | PCT/RU 02/00507 |

| | |
|---|---|
| A.   CLASSIFICATION OF SUBJECT MATTER      B 01 J 23/74, 23/89, C 07 C 1/04 | |

According to International Patent Classification (IPC) or to both national classification and IPC  7

| B.   FIELDS SEARCHED |
|---|

Minimum documentation searched (classification system followed by classification symbols)  IPC 7

B 01 J 23/74, 23/89, C 07 C 1/04

Documentation searched other than minimum documentation to the extent that such documents are included in the fields searched

Electronic data base consulted during the international search (name of data base and, where practicable, search terms used)

| C.   DOCUMENTS CONSIDERED TO BE RELEVANT | | |
|---|---|---|
| Category* | Citation of document, with indication, where appropriate, of the relevant passages | Relevant to claim No. |
| A | RU 2161067 C2 (ADZHIN  PETROLI S.P.A. et al), 27.12.2000 | 1-13 |
| A | EP 0857513 A1 (AGIP PETROLI S.P.A.), 12.08.1998; | 1-13 |
| A | US 5169821 A (EXXON RESEARCH AND ENGINEERING COMPANY), 08.12.1992; | 1-13 |
| A | EP 0510770 A2 (SHELL INTERNATIONALE RESEARCH MAATSCHAP-PIJ B.V.), 28.10.1992 | 1-13 |

☐   Further documents are listed in the continuation of Box C.      ☐      See patent family annex.

| | |
|---|---|
| *      Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier document but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | |
| | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| | |
|---|---|
| Date of the actual completion of the international search | Date of mailing of the international search report |
| 17 February 2003 (17.02.03) | 20 February 2003 (20.02.03) |
| Name and mailing address of the ISA/  RU | Authorized officer |
| Facsimile No. | Telephone No. |

Form PCT/ISA/210 (second sheet) (July 1992)